# EUROPÄISCHE PATENTSCHRIFT

(11) **EP 1 290 063 B1**
(45) Veröffentlichungstag und Bekanntmachung des Hinweises auf die Patenterteilung: **16.11.2005**
(21) Anmeldenummer: 01936410.8
(22) Anmeldetag: 30.05.2001
(51) Int. Cl.: C08G 77/46, C08G 77/388, A61K 6/10, A61K 6/093, C08L 83/12

(54) **N-ALKYL-AZIRIDINOBLOCKCOPOLYMERE UND DEREN VERWENDUNG**
N-ALKYL AZIRIDINE BLOCK COPOLYMERS AND THE USE THEREOF
COPOLYMERES SEQUENCES N-ALKYLAZIRIDINO ET LEUR UTILISATION

(30) Priorität: 31.05.2000 DE 10026852
(43) Veröffentlichungstag der Anmeldung: 12.03.2003
(73) Patentinhaber: 3M ESPE AG, 82229 Seefeld (DE)
(72) Erfinder: NOWAK, Reinhold, 82276 Adelshofen (DE); ZECH, Joachim, 86916 Kaufering (DE); BISSINGER, Peter, 86911 Diessen (DE); WANEK, Erich, 86916 Kaufering (DE); ECKHARDT, Gunther, 06231 Bad Dürrenberg (DE); LECHNER, Günther, 82237 Wörthsee (DE)
(86) Internationale Anmeldenummer: PCT/EP2001/006141
(87) Internationale Veröffentlichungsnummer: WO 2001/092374

(56) Entgegenhaltungen:
- EP-A- 0 982 041
- WO-A-00/47165
- US-A- 3 159 600
- US-A- 5 569 691
- PATENT ABSTRACTS OF JAPAN vol. 009, no. 237 (C-305), 24. September 1985 (1985-09-24) & JP 60 094486 A (DAINIPPON INSATSU KK;OTHERS: 01), 27. Mai 1985 (1985-05-27) & DATABASE WPI/DERWENT [Online] An 1985-163004,

## Beschreibung

Die Erfindung betrifft N-Alkyl-Aziridinoblockcopolymere und deren Verwendung, insbesondere in dentalen Zubereitungen.

Unter N-Alkyl-Aziridinoblockcopolymeren im Sinne dieser Erfindung sind ölige oder harzartige Polymere gemeint, die über einen Silikon-Kemblock verfügen, der mit mehreren Polyetherblöcken verbunden ist, an deren jeweilig anderem Ende wiederum Aziridinogruppen angeknüpft sind.

Hochpräzise elastische Abformmaterialien, die sich durch hohe Abformgenauigkeit, hohe Formbeständigkeit und gute Detailwiedergabe auszeichnen, sind beispielsweise Materialien auf der Basis von Agar-Agar, Polysulfiden, Polyethern oder additionsvernetzenden Silikonen.

Bei den additionsvernetzenden Silikonabformmaterialien wird die Härtung durch Reaktion eines Polysiloxans mit Vinylendgruppen mit einem Polysiloxan mit Si-H-Gruppen mittels Platinkatalysatoren erreicht. Die so erhaltenen Abdrücke zeichnen sich durch sehr gute elastische Eigenschaften und hohe Lagerbeständigkeiten aus. Nachteilig bei diesen Materialien ist schon immer die geringe Hydrophilie gewesen, die zu geringer Zeichnungsschärfe aufgrund eines schlechten Anfließvermögens führt.

Zur Verbesserung des hydrophilen Verhaltens von Silikonabdruckmaterialien werden den additionsvemetzenden Silikonabdruckmaterialien hydrophilierende Zusätze hinzu gefügt. Die so erzielte bessere Benetzbarkeit ist jedoch auch mit einer erhöhten Wasseraufnahme beim Kontakt mit feuchten Medien verbunden, was eine verschlechterte Dimensionsstabilität und verstärkte Wasserstoffentwicklung zur Folge haben kann.

Bei den reinen Polyethermaterialien werden aziridinhaltige Substanzen polymerisiert, wie sie in den US-A-34 53 242 und 40 93 555 oder auch in der DE-A-43 06 997 beschrieben sind. Zur Initiierung der Polymerisation sind beispielsweise die aus der US-A-41 67 618 bekannten Sulfoniumsalze gut geeignet. So hergestellte Polyetherabformmaterialien weisen natürliche hydrophile Eigenschaften auf.

Es gab verschiedene Ansätze, die hydrophilen Eigenschaften der Polyether mit den guten elastischen Eigenschaften der Silikone zu kombinieren:

In der DE-A-37 41 575, DE-A-40 19 249, DE-A-40 10 281 sowie in der DE-A-38 38 587 sind Massen auf der Basis einer platinkatalysierten Additionsreaktion einer Si-H-Komponente mit einem ungesättigten Polyether beschrieben. Der ungesättigte Polyether ist im Gegensatz zu additionsvemetzenden Silikonen in der Regel der Hauptbestandteil, welcher der Matrix eine hydrophile Charakteristik verleiht.

In der DE-A-40 19 249 sind härtbare Massen beschrieben, die neben ungesättigten Polyethern mit endständigen Alkenylresten auch die Umsetzungsprodukte solcher substituierter Polyether mit Oligosiloxanen mit mindestens zwei Si-H-Gruppen im Molekül sowie Platinkatalysatoren als Hauptbestandteile enthalten.

Zur Erlangung ausreichender Lagerfähigkeit ist es notwendig, die reaktiven Bestandteile räumlich voneinander zu trennen. Hierbei können die Si-H-Verbindung und der für eine Aushärtung bei Raumtemperatur erforderliche Platinkatalysator nicht in einer Paste vereinigt werden, da es zur Zersetzung der Si-H-Verbindung kommt.

Im Zuge einer Lagerung über einen Zeitraum von mehreren Wochen bis Monaten zeigt sich jedoch das Problem, dass auch eine Katalysatorkomponente, bei der der Platinkatalysator mit dem ungesättigten Polyether zusammen vorliegt, eine nicht befriedigende Lagerstabilität aufweist.

Für den Zahnarzt oder den Techniker besteht allerdings die Notwendigkeit, eine Abformmasse zur Verfügung zu haben, die lagerfähig ist und deren Anwendbarkeit über einen Zeitraum von mehreren Monaten bis Jahren gewährleistet ist.

In DE-A-40 10 281 wird daher ein Zusatz von Antioxidantien zur Erhöhung der Lagerstabilität vorgeschlagen. Jedoch wird auch dadurch nur eine nicht zufriedenstellende Langzeit-Lagerstabilität erreicht.

In der DE-A-197 19 438 werden additionsvemetzende Polyether-Abformmaterialien beschrieben, die sich durch eine gute Lagerstabilität der Katalysatorkomponente sowie der Basiskomponente als auch der ausgehärteten Dentalmasse auszeichnen. Nachteilig an diesen Dentalmassen ist das niedrige Niveau der mechanischen Eigenschaften, so dass sie nur eingeschränkt für die dentale Abformung einsetzbar sind.

Aufgabe der vorliegenden Erfindung ist es, Massen bereitzustellen, die die Nachteile des Standes der Technik nicht aufweisen.

Diese Aufgabe wurde gelöst durch N-Alkylaziridinoblockcopolymere als Basis für die Herstellung von härtbaren Massen, die in den Ansprüchen beschrieben sind.

Die Einzelkomponenten der daraus hergestellten Massen weisen eine gute Lagerbeständigkeit auf. Beispielsweise wird bei der Verwendung als Dentalmassen eine hohe Abformgenauigkeit erzielt. Im ausgehärteten Zustand zeichnen sich diese durch gute mechanische Eigenschaften aus.

Die erfindungsgemäß eingesetzten N-Alkylaziridinoblockcopolymere haben die nachfolgend dargestellten allgemeinen Strukturen gemäß Formel (1): 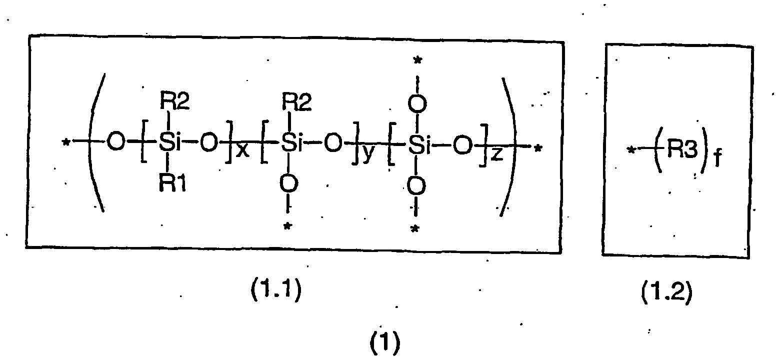 wobei bedeuten:
- R1 =: H, C₁-C₁₂-Alkyl, C₂-C₁₂-Alkenyl, C₂-C₁₂-Alkinyl, C₇-C₁₅-Alkaryl, C₇-C₁₅-Aralkyl oder C₃-C₁₂-Cycloalkyl und diese Reste teilweise, ganz oder gemischt mit Cl oder F substituiert sein können und/oder 0 bis 5 Heteroatome aus der Gruppe O, N, S enthalten können; bevorzugt sind: H, Methyl, Ethyl, Ethenyl, Propenyl, Phenyl, Tolyl, 2-Ethylphenyl, Cyclohexenyl;
- R2 =: einen Rest aus der Gruppe R1 und/oder R4;
- R3=: SiR1₃ oder SiR1₂R4
mit:
R4 = ein Vertreter der allgemeinen Formel (2): 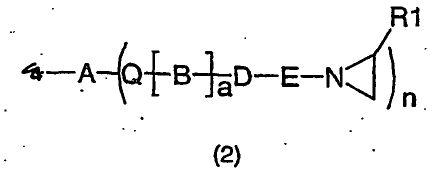 worin
A = ein (n + 1)-fach radikalischer gesättigter, ungesättigter oder aromatischer, linearer, verzweigter oder cyclischer Kohlenwasserstoffrest, der 0 bis 5 Heteroatome aus der Gruppe O, N, S enthalten kann und 1 bis 18 Kohlenstoffatome, bevorzugt 1 bis 12 Kohlenstoffatome umfasst;
B = ein Vertreter der Gruppe: O, S, NR1;
D = ein Vertreter der Gruppe: C(O)O, C(O)NR1, C(O), C(S)NR1, CH₂;
E = ein 2-fach radikalischer gesättigter oder ungesättigter, linearer, verzweigter oder cyclischer Kohlenwasserstoffrest, der 0 bis 5 Heteroatome aus der Gruppe O, N, S, enthalten kann und 0 bis 18 Kohlenstoffatome, bevorzugt 1 bis 12 Kohlenstoffatome umfasst;
Q = eine α, ω- radikalische Polyetherkette der allgemeinen Formel (3): 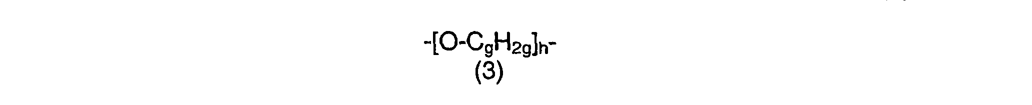 wobei bedeuten:
g = 2 bis 20, bevorzugt 2 bis 10, besonders bevorzugt 2 bis 7;
h = 1 bis 1000, bevorzugt 1 bis 500, besonders bevorzugt 1 bis 200;
a = 0 oder 1;
f = eine ganze Zahl von 2 bis 1000, bevorzugt 2 bis 100, besonders bevorzugt 2 bis 50;
n = eine ganze Zahl von 1 bis 10, bevorzugt 1 bis 5, besonders bevorzugt 1 bis 3;
sowie
- x, y, z :: jeweils entweder 0 oder ganze Zahlen darstellen, deren Summe zwischen 1 und 10.000, bevorzugt zwischen 1 und 1.000 und besonders bevorzugt zwischen 10 und 500 liegen soll, wobei die mittlere Molmasse Mₙ der Produkte bevorzugt zwischen 500 und 50.000 und besonders bevorzugt zwischen 1.000 und 20.000 liegen kann,
mit den Einschränkungen, dass, wenn x größer 0 ist, y oder z kleiner oder gleich x, bevorzugt kleiner oder gleich 0,05 mal x und besonders bevorzugt 0,02 mal x sein soll.

Das Symbol "*" in der Formel (1.2) bedeutet, dass die so markierte Valenz mit den mit "*" markierten Positionen des Fragments (1.1) verknüpft wird.

Von den jeweils x Resten von R2 können bis zu 0,5 mal x Reste R4 bedeuten, die anderen bedeuten R1.

Im Fall von y + z = 0 liegen lineare Polysiloxane, im Fall von y + z > 0 verzweigte Polysiloxane vor, wobei Formel (1) insofern zu verstehen ist, als das Polymer in diesem Falle sowohl D-, als auch T- und / oder Q-Einheiten im Sinne der SilikonNomenklatur enthalten kann, die im Molekül an beliebiger Stelle stehen können und an den Anknüpfungspunkten der T- und Q-Einheiten beliebig lange D-Ketten aufweisen deren summarische Länge x D-Einheiten beträgt.

Jede Nennung des Restes R1 bedeutet lediglich die Entnahme aus der unter R1 getroffenen Auswahl, verschiedene Nennungen können an jedem unterschiedlichen Substitutionspunkt und auch an jeder Wiederholungseinheit einer polymeren Formel, unterschiedliche Reste R1 bedeuten. Das bedeutet, dass mit dem Faktor - (SiOR1R2)ₓ- sowohl Homopolymere mit einem definierten R1 und R2 als auch Copolymere von Silikonen unterschiedlicher Reste R1 und R2 gemeint sind, wobei x jedoch die Anzahl aller damit umfassten Siliczium-Atome ohne Einbeziehen der getroffenen Auswahl an Resten R1 und R2 bedeutet. Analoges gilt sinngemäß auch für den Faktor -(SiR2(O*))_{y}- und R3.

Im allgemeinen sind keine polymereinheitlichen Polysiloxan-Grundketten vorhanden. In Abhängigkeit vom Herstellungsverfahren kann die Polydispersität (M_{w}/Mₙ) 1,1 bis 20 und bevorzugt 1,2 bis 10 betragen.

In Abhängigkeit vom Herstellungsverfahren kann die Polydispersität der Polyetherblöcke 1,1 bis 20 und bevorzugt 2 bis 10 betragen.

Der Polyetherblock kann ein Homopolymer, ein Copolymer oder auch ein Terpolymer sein. Die Mischpolymerisate können altemierend oder statistisch aufgebaut sein bzw. alternierende und statistische Mischpolyetherblöcke besitzen, die ggf. mit Homopolymer-Polyetherblöcken verbunden sind.

Bevorzugte Polyetherblöcke sind Polytetrahydrofuran, Polypropylenoxid, statistische Copolymere aus Ethylenoxid und Tetrahydrofuran, aus Propylenoxid und Tetrahydrofuran, aus Ethylenoxid und Propylenoxid, Blockcopolymere aus Ethylenoxid und Propylenoxid und statistische Terpolymere aus Ethylenoxid, Propylenoxid und Tetrahydrofuran.

Bevorzugte Vertreter der Formel (2) sind, nachstehend aufgeführt, wobei neben den N-Alkyl-ethylenimin-Derivaten auch die N-Alkyl-propylenimin-Derivate gemeint sind: 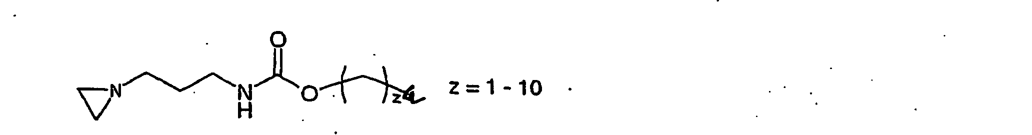 aus: A = (CH₂)_{z}; B = O; D = C(O)NR1 (mit R1 = H); E = 1,3-propandiyl; a = 1; n=1 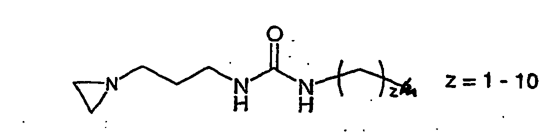 aus: A = (CH₂)_{z}; B = NH; D = C(O)NR1 (mit R1 = H); E =1,3-propandiyl; a=1;n=1 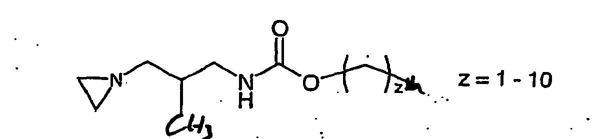 aus: A = (CH₂)_{z}; B = O; D = C(O)NR1 (mit R1 = H);
E = 2-methyl-1,3-propandiyl; a =1; n = 1 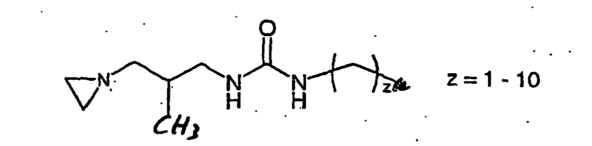 aus: A = (CH₂)_{z}; B = NH; D = C(O)NR1 (mit R1 = H);
E = 2-methyl-1,3-propandiyl; a = 1; n = 1 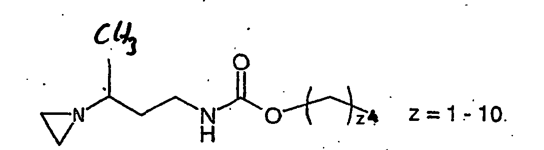 aus: A = (CH₂)_{z}; B = O; D = C(O)NR1 (mit R1 = H); E = 1,3-butandiyl; a = 1; n = 1 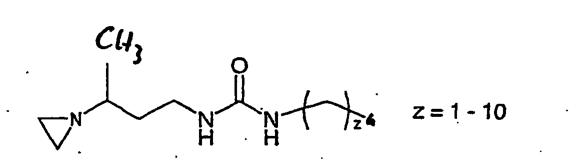 aus: A = (CH₂)_{z}; B = NR1 (R1 = H); D = C(O)NR1 (mit R1 = H);
E = 1,3-butandiyl; a = 1; n = 1 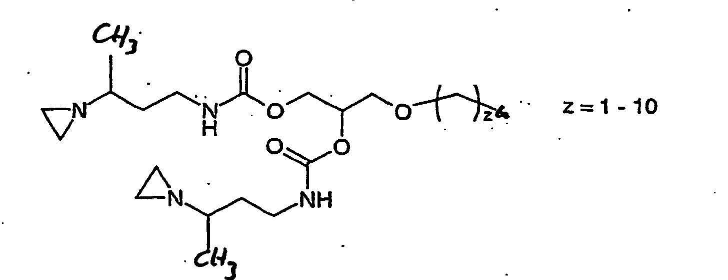 aus: A = 3-oxa-heptan-1,2,7-triyl (für z=3); B = O; D = C(O)NR1 (mit R1 = H);
E = 1,3-butandiyl; a = 1; n = 2 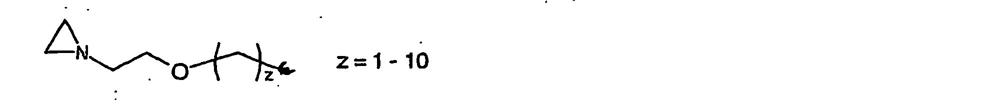 aus: A = (CH₂)_{z}; B = O; D = CH₂; E = methandiyl; a =1; n = 1 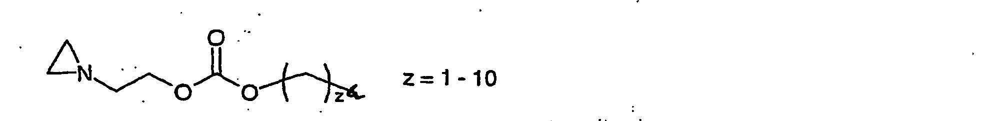 aus: A = (CH₂)_{z}; B = O; D = C(O)O; E = 1,2-ethandiyl; a = 1; n = 1 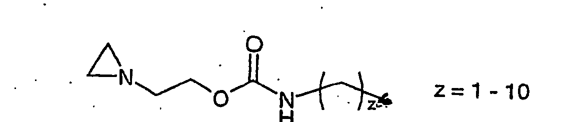 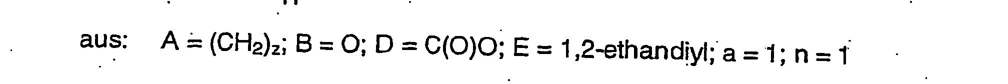 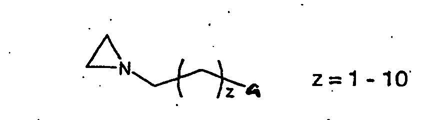 aus: A = (CH₂)_{z+1}; D = CH₂; E = CH₂; a = 0; n = 1 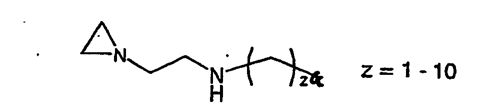 aus: A = (CH₂)_{z}; B = NH; D = CH₂; E = 1,2-ethandiyl; a =1; n = 1 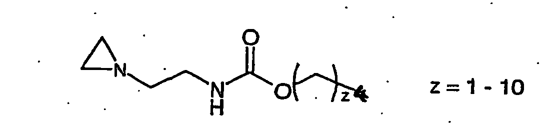 aus: A = (CH₂)_{z}; B = NH; D = C(O)O; E = 1,2-ethandiyl; a = 1; n = 1 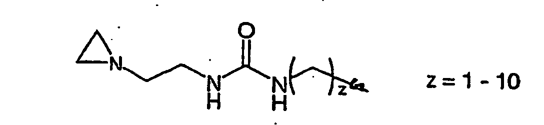 aus: A = (CH₂)_{z}; B = NH; D = C(O)NH; E = 1,2-ethandiyl; a = 1; n = 1 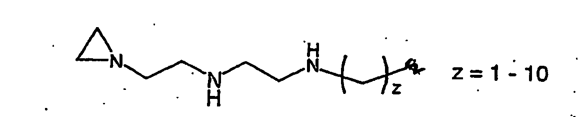 aus: A = (CH₂)_{z}; B = NH; D = CH₂; E = 2-aza-1,4-butandiyl; a =1; n = 1 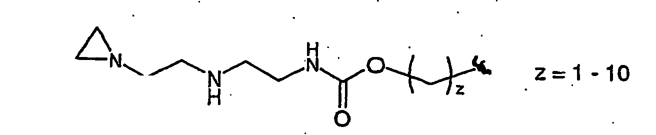 aus: A = (CH₂)_{z}; B = NH; D = C(O)O; E = 2-aza-1,4-butandiyl; a = 1; n = 1 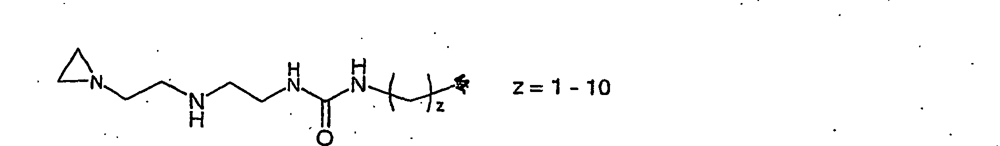 aus: A = (CH₂)_{z}; B = NH; D = C(O)NH; E = 2-aza-1,4-butandiyl; a = 1; n = 1 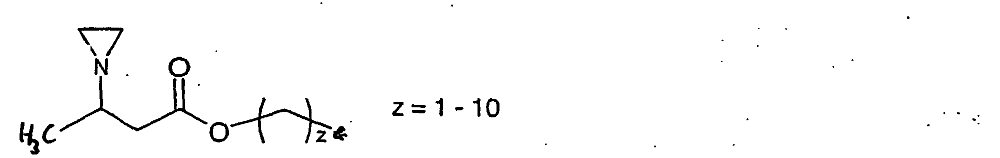 aus: A = (CH₂)_{z}; B = O; D = C(O); E = 2-methyl-1,2-propandiyl; a = 1; n = 1 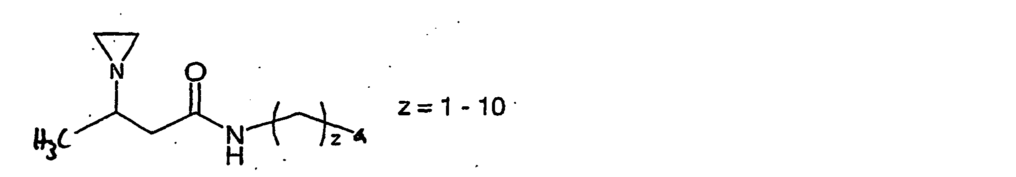 aus: A = (CH₂)_{z}; B = NH; D = C(O); E = 2-methyl-1,2-propandiyl; a =1; n = 1 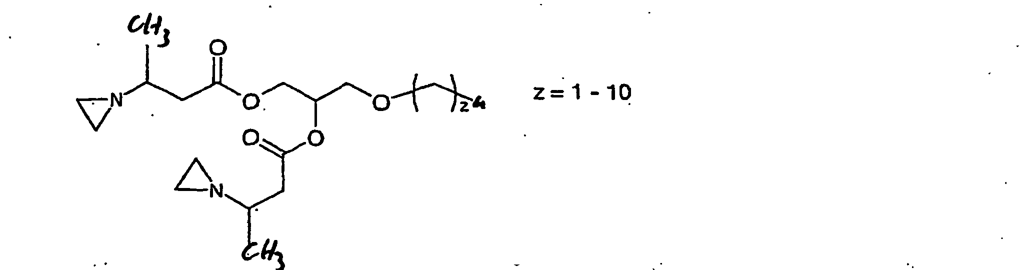 aus: A = 3-oxa-heptan-1,2,7-triyl; B = O; D = C(O);
E = 2-methyl-1,2-propandiyl; a = 1; n = 2 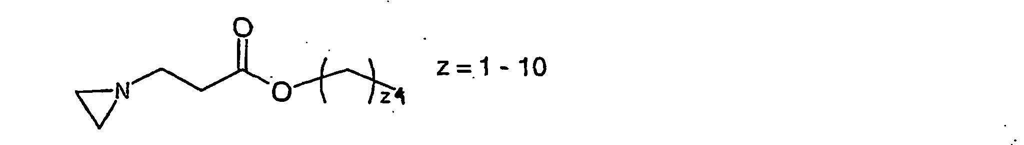 aus: A = (CH₂)_{z}; B = O; D = C(O); E = 1,2-ethandiyl; a = 1; n = 1 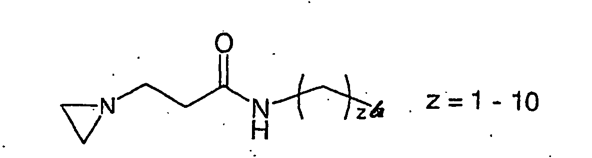 aus: A = (CH₂)_{z}; B = NH; D = C(O); E = 1,2-ethandiyl; a = 1; n = 1 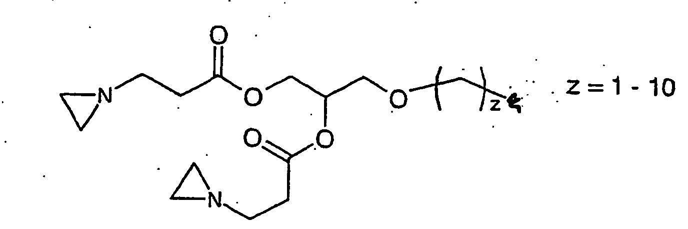 aus: A = 3-oxa-heptan-1,2,7-triyl; B = O; D = C(O); E = 1,2-ethandiyl; a = 1; n = 2 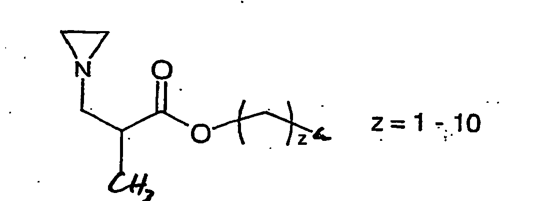 aus: A = (CH₂)_{z}; B = O; D = C(O); E =1-methyl-1,2-propandiyl; a = 1; n = 1 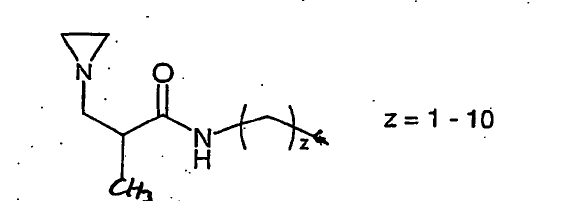 aus: A = (CH₂)_{z}; B = NH; D = C(O); E = 1-methyt-1,2-propandiyl; a = 1; n = 1 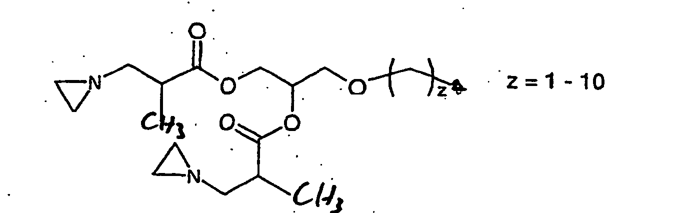 aus: A = 3-oxa-heptan-1,2,7-triyl; B = O; D = C(O);
E = 1,-methyl-1,2-propandiyl; a = 1; n = 2

Die erfindungsgemäßen N-Alkylaziridinoblockcopolymere können Molmassen im Bereich von 500 bis 50.000 g / Mol, bevorzugt von 1000 bis 30.000 g / Mol und besonders bevorzugt im Bereich von 3000 bis 20.000 g / Mol aufweisen. Sie besitzen mindestens eine und vorzugsweise bis zu zehn N-Alkylaziridinogruppen im Molekül. Die Verwendung von Gemischen von N-Alkylaziridinoblockcopolymeren mit unterschiedlicher Molmasse und unterschiedlicher Anzahl von N-Alkylaziridinogruppen ist möglich und wird zur Einstellung der Eigenschaften von daraus formulierten Massen genutzt.

Zur Einstellung der jeweils gewünschten Netzwerkstruktur können die erfindungsgemäßen N-Alkylaziridinoblockcopolymere stark unterschiedliche Aziridinoäquivalentmassen besitzen, wobei der Bereich von 250 bis 25.000 g/Äquivalent und besonders von 400 bis 10.000 g/Äquivalent bevorzugt ist.

Prinzipiell wird bei der Herstellung der erfindungsgemäßen N-Alkylaziridinoblockcopolymere so verfahren, dass ein käuflich erhältliches funktionalisiertes Polysiloxan in einem oder mehreren Schritten zum Aziridino-BlockCopolymeren umgesetzt wird. Statt die käuflich erhältlichen funktionalisierten Polysiloxane als solche einzusetzen, können diese auch in sich, als Mischung und/oder als Mischung mit Silikonrohstoffen durch Äquilibrierung, Polymerisation, Copolymerisation, Depolymerisation etc. in bekannter Art (siehe P. Kochs in Houben-Weyl, Bd. E20, S. 2219 ff.) verändert und erst danach zum Aziridino-BlockCopolymeren funktionalisiert werden. Eine Vielzahl von bevorzugten Vertretern solcher funktionalisierten Silikonöle können beispielsweise im Katalog "Reactive Silicones" der Firma Gelest gefunden werden. Sie können jedoch auch nach literaturbekannten Vorschriften selbst hergestellt werden, beispielsweise gemäß H. R. Kricheldorf (Ed.) "Silicon in Polymer Synthesis" Springer 1996, Chapt. 3. Besonders geeignet zur Funktionalisierung sind Hydrido-, Hydroxyalkyl- und Aminoalkyl-funktionalisierte Siloxane.

Solche funktionalisierten Silikone können dann auf verschiedenen Wegen zu den erfindungsgemäßen Polyether-Silikon-Blockcopolymeren umgewandelt werden. Bevorzugt sind aus Stabilitätsgründen dabei Blockcopolymere, bei denen Verknüpfungen über hydrolytisch instabile Si-O-C-Bindungen zugunsten von hydrolytisch stabilen Si-C-Bindungen vermieden werden. Blockcopolymere mit Si-O-C-Bindungen sind dagegen aus Kostengründen bevorzugt, da hier von den sehr preisgünstigen Silanol-terminierten Silikonölen ausgegangen werden kann. Die Anknüpfung von Polyetherblöcken kann beispielsweise durch kationische Pfropfpolymerisation von cyclischen Ethem oder Gemischen solcher auf Silikonkörper mit protischen Endgruppen erfolgen. Eine andere Möglichkeit ist die Verknüpfung von Chlorformiaten, Chloroxalaten oder andere Halbester/Halbamidsäurechloriden solcher Silikonkörper mit Polyetherdiolen. Eine weitere Möglichkeit ist die Hydrosilylierung von Polyethermonoallylethern an hydridofunktionalisierte Silikonöle. Durch Hydrosilylierung an Silikonmonomere wie M^{H}₂ oder D^{H}ₘ (nach gängiger Silikonnomenklatur: Encylopedia of Polymerscience and Engineering 2. Ed., Bd. 15, S. 206 f.; H = Hydrido-funktionalisiert) und anschließender Äquilibrierung mit einem Silikonkörper oder Copolymerisation, beispielsweise mit D₄, kann das Ziel ebenfalls erreicht werden. Diese und weitere Silikon-Polyether-Block-Copolymere sind nach Vorschriften in J. B. Plumb et al. "Block Copolymers" Applied Science Pub., 1973, S. 306 ff. darstellbar.

Aziridinoverbindungen können aus den Blockcopolymeren, beispielsweise über Chloroxalate oder Chlorformiate hergestellt werden, wobei in manchen Fällen aus den Chloroxalaten oder -formiaten intermediär aktivierte Amide, wie Imidazolide hergestellt werden. Als Aziridinokomponente kommen beispielsweise Aziridinoethanol (Acros) oder andere hydroxy- oder aminofunktionelle Aziridinoverbindungen in Frage.
Neben diesen Methoden zur Synthese von Aziridinoverbindungen finden sich in den folgenden Monographien sowie in den darin zitierten Literaturstellen eine Vielzahl von möglichen Synthesevarianten zum Aufbau und zur Derivatisierung oder Modifizierung von Aziridinoverbinungen. Die bevorzugten Vertreter der Aziridinosilikone sowie nötigenfalls deren Vorstufen sind nach den dort beschriebenen Vorgehensweisen darstellbar, wobei einige dieser Methoden vom Fachmann sinnvoll anzupassen sind: R.C. Elderfield, "Heterocyclic Compounds" Vol. 1, S. 61-77 Wiley 1950; Houben-Weyl "Methoden der Organischen Chemie", Bd. XI/2 S. 223-264, Thieme 1958; O.C. Dermer, G.E. Ham, "Ethylenimine and other Aziridines", insbesondere S. 106-205 sowie S. 340-393, Academic Press 1969; Houben-Weyl "Methoden der Organischen Chemie", Bd. E16c S. 370-667, Thieme 1992; Ulmanns Encyclopedia of Industrial Chemistry 5^{th} Ed. Vol. A3 S. 239-243.

Weiterhin sind Gegenstand der Erfindung härtbare Massen auf der Basis der erfindungsgemäßen N-Alkylaziridinoblockcopolymeren, insbesondere zweikomponentige Dentalmassen, wobei diese nach der Mischung der Komponenten, jeweils bezogen auf 100 Masseteile, enthalten:
(A) 30 bis 97, bevorzugt 40 bis 89, besonders bevorzugt 45 bis 80,5 Masseteile von mindestens einem N-Alkylaziridinoblockcopolymer mit Molmassen im Bereich von 500 bis 50.000 g/Mol und Aziridinoäquivalentmassen im Bereich von 250 bis 25.000 g/Äquivalent
(B) 1 bis 10, bevorzugt 1 bis 5, besonders bevorzugt 1,5 bis 3 Masseteile von Startersubstanzen, die geeignet sind, die Aushärtung der N-Alkylaziridinoblockcopolymeren zu bewirken,
(C) 1 bis 35, bevorzugt 5 bis 25, besonders bevorzugt 8 bis 20 Masseteile von organischen Verdünnungsmitteln,
(D) 1 bis 50, bevorzugt 5 bis 40, besonders bevorzugt 10 bis 30 Masseteile von Modifikatoren, einschließlich Füllstoffen, Farbstoffen, Pigmenten, Thixotropiemitteln, Fließverbesserem, polymeren Eindickern, oberflächenaktiven Substanzen, Geruchsstoffen und Geschmacksstoffen.

Die durch homogene Mischung der Katalysatorkomponente und der Basiskomponente erzeugte Zubereitung besitzt beispielsweise eine Verarbeitungszeit bei Raumtemperatur zwischen 0,5 bis 10 Minuten und die so hergestellte Zubereitung härtet beispielsweise in einem Temperaturbereich von 23 bis 36° C innerhalb einer Zeitspanne von einer bis 20 Minuten zu einer elastisch deformierbaren Masse mit einer Shore A-Härte von mindestens 20 aus.

Im Falle von zweikomponentigen Massen werden die Bestandteile (A) bis (D) vor der Mischung auf die Basis- und die Katalysatorkomponente so aufgeteilt, dass der Bestandteil (A) vollständig in der Basiskomponente und der Bestandteil (B) vollständig in der Katalysatorkomponente enthalten ist. Die Bestandteile (C) und (D) können anteilig in den Komponenten vorliegen.

Das Mischungsverhältnis kann in einem weiten Bereich über die Zusammensetzung der beiden Komponenten eingestellt werden, wobei sich Mischungsverhältnisse von Katalysator- zu Basiskomponente von 1 : 1 bis 1 : 5 als besonders praktikabel erwiesen haben.

Die Anteile der einzelnen Bestandteile (A) bis (D) sind innerhalb der angegebenen Grenzen so einzustellen, dass eine günstige Verarbeitbarkeit hinsichtlich Mischungsverhältnis und Fließverhalten gewährleistet ist und die Kriterien für die gewünschte Verarbeitungszeit, die Aushärtungszeit und die mechanischen Eigenschaften der ausgehärteten Masse eingehalten werden.

Bestandteil (A) enthält die erfindungsgemäßen N-Alkylaziridinoblockcopolymere. Die Verwendung von Gemischen von N-Alkylaziridinoblockcopolymeren mit unterschiedlichen Molmassen und Aziridinoäquivalentmassen ist möglich und wird zur Einstellung der Eigenschaften der Massen genutzt.

Zur Einstellung der gewünschten mechanischen Eigenschaften der ausgehärteten Massen können die erfindungsgemäßen Zubereitungen Verbindungen mit Polyetherstruktur ohne Silikonblöcke und mit mindestens einer und bevorzugt zwei N-Alkylaziridinogruppen enthalten.

Diese zusätzlich verwendbaren N-Alkylaziridinopolyether können Aziridinoäquivalentmassen von 250 bis 10000 g / Äquivalent besitzen, wobei die Polyether-Grundkörper Homopolymere aus Ethylenoxid, Propylenoxid oder Tetrahydrofuran, statistische Co- und Terpolymere der genannten Monomeren und bzw. oder Blockcopolymere aus Ethylenoxid und Propylenoxid sein können.

Als Startersubstanz gemäß Bestandteil (B) der gemischten Zubereitung kommen eine Reihe von Verbindungen in Betracht, wenn sie die Kriterien hinsichtlich Abbindegeschwindigkeit und resultierenden Elastomereigenschaften erfüllen.

So sind für die Verwendung in zweikomponentigen Abformmassen, die auf den vorstehend beschriebenen Polyetherderivaten beruhen, solche Startersubstanzen geeignet, die eine Aushärtung der gemischten Zubereitung in einem Zeitraum von 1 bis 20 Minuten zu einem elastischen Festkörper ermöglicht, wobei dieser Festkörper die Anforderungen an eine elastische Abformmasse gemäß DIN / EN 2482 erfüllt und eine Shore A-Härte (DIN 53505) von mindestens 20 nach 24 Stunden Lagerzeit besitzt.

Als Starter der Katalysatorkomponente können viele der bekannten Starter eingesetzt werden. Zweckmäßigerweise verwendet man solche Starter bzw. Startersysteme, die eine einfache Einstellung des Aushärtungsverlaufs zulassen, keine Nebenwirkungen erzeugen und die reproduzierbare Erreichung der erforderlichen Niveaus der mechanischen Eigenschaften ermöglichen.

In der DE-C-914 325 wird die Verwendung von Oxonium-, Ammonium- und Sulfoniumsalzen als Startersubstanzen vorgeschlagen.

Eine zusammenfassende Darstellung der für die Aushärtung von N-Alkylaziridinoverbindungen verwendeten Startersubstanzen ist in O. C. DERMER, G. E. HAM, "Ethylenimine and other Aziridines" Academic Press (1969) enthalten.

Als prinzipiell geeignete Polymerisationsauslöser haben sich demnach eine große Anzahl von Verbindungsklassen und Verbindungen erwiesen. In der praktischen Anwendung der kationischen Polymerisation von Aziridinopolyethern ist es aber sehr schwierig, den gewünschten Abbindeverlauf mit ausreichend langer Verarbeitungszeit und schneller Endaushärtung einzustellen. Dieses Ziel kann durch die Verwendung von speziellen Trisalkylsulfoniumsalzen erreicht werden, wie sie beispielsweise in der EP-A-0 110 429 beschrieben sind.

Unter Verwendung von speziellen Trisalkylsulfoniumsalzen sind die Kriterien der die Härtungsgeschwindigkeit und der Eigenschaften des elastischen Festkörpers prinzipiell erreichbar.

In der Patentanmeldung DE-100 18 918 werden Starter beschrieben, die der Katalysatorkomponente einen lediglich geringen Säuregrad verleihen und die eine gut einstellbare, relativ lange Verarbeitungszeit nach erfolgter Mischung von Basiskomponente und Katalysatorkomponente ermöglichen.

Startersysteme dieses Typs sind geeignet, die erfindungsgemäßen Basispasten in der notwendigen Geschwindigkeit auszuhärten. Durch ihre Verwendung sind die gewünschten Eigenschaften des elastischen Festkörpers erreichbar.

Die Patentanmeldung DE-19942459 beschreibt Elastomermassen mit verbesserter Katalysatorkomponente, die sich durch eine erhöhte Dehnbarkeit auszeichnen. Gemäß dieser Erfindung werden Borsäurekomplexe als Starter eingesetzt. Diese Starter haben sich für die Aushärtung der N-Alkylaziridinopolyether gemäß vorliegender Erfindung besonders bewährt und werden mit Vorteilen gegenüber anderen Startersystemen eingesetzt.

Als organisches Verdünnungsmittel entsprechend Bestandteil (C) werden Polyetherpolyole, wie Polypropylenglykole oder Mischpolyetherole mit Tetrahydrofuran- und/oder Ethylenoxid- und/oder Propylenoxid-Einheiten, Polyesterpolyole, wie Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle Ester von mehrwertigen Säuren, wie Phthalsäure oder Zitronensäure oder Ester oder Amide von Alkylsulfonsäuren und Arylsulfonsäuren verwendet.

Weiterhin sind flüssige Organosiloxane, wie Polydimethylsiloxane unterschiedlicher Kettenlänge einsetzbar.

Weitere gemäß diesem Bestandteil einzusetzende Verbindungen genügen den nachfolgenden allgemeinen Strukturen: 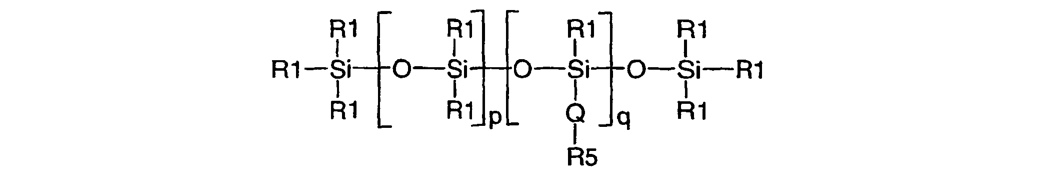 und / oder 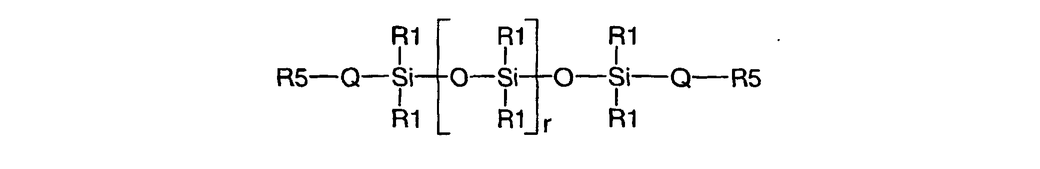 wobei bedeuten:
- Q =: eine α, ω-radikalische Polyetherkette der Formel (3):

-[O-C_{g}H_{2g}]ₕ- (3);
- R5 =: OH, OR1, O-C(O)-R1, O-C(O)-NHR1;
- g =: 2 bis 20, bevorzugt 2 bis 10, besonders bevorzugt 2 bis 7;
- h =: 1 bis 1000, bevorzugt 1 bis 500, besonders bevorzugt 1 bis 200;
- a =: 0 oder 1;
- f =: eine ganze Zahl von 0 bis 1000, bevorzugt 1 bis 100, besonders bevorzugt 1 bis 50;
- n =: eine ganze Zahl von 1 bis 10, bevorzugt 1 bis 5, besonders bevorzugt 1 bis 3;
- p =: eine ganze Zahl von 1 bis 1000, bevorzugt 3 bis 500;
- q =: eine ganze Zahl von 1 bis 50, bevorzugt 3 bis 10;
- r =: eine ganze Zahl von 1 bis 1000, bevorzugt 1 bis 500.

In Abhängigkeit vom Herstellungsverfahren kann die Polydispersität der Polyetherblöcke 1,1 bis 20 und bevorzugt 2 bis 10 betragen.

Der Polyetherblock kann ein Homopolymer, ein Copolymer oder auch ein Terpolymer sein. Die Mischpolymerisate können alternierend oder statistisch aufgebaut sein bzw. altemierende und statistische Mischpolyetherblöcke besitzen, die ggf. mit Homopolymer-Polyetherblöcken verbunden sind.

Bevorzugte Polyetherblöcke sind Polytetrahydrofuran, Polypropylenoxid, statistische Copolymere aus Ethylenoxid und Tetrahydrofuran, aus Propylenoxid und Tetrahydrofuran, aus Ethylenoxid und Propylenoxid, Blockcopolymere aus Ethylenoxid und Propylenoxid und statistische Terpolymere aus Ethylenoxid, Propylenoxid und Tetrahydrofuran.

Der Katalysatorkomponente wie auch der Basiskomponente können Modifikatoren, entsprechend dem Bestandteil (D) in einem weiten Konzentrationsbereich zugesetzt werden.

Diese Modifikatoren sind meist feinteilige Füllstoffe, wie Alumosilikate, Fällungskieselsäuren, Quarzmehl, Wollastonit, Glimmermehl und Diatomeenerde, sowie Farbstoffe und Pigmente, deren Zusatz eine bessere Beurteilung der Mischgüte ermöglicht und die Verwechslungsgefahr vermindert, Thixotropiemittel, wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchsstoffe und Geschmacksstoffe.

Die erfindungsgemäßen Zubereitungen können in Abhängigkeit von der Zusammensetzung der Katalysatorkomponente und der Basiskomponente für das Verkleben von Substraten, für das Abdichten, das Beschichten und das Vergießen eingesetzt werden.

Die erfindungsgemäßen Massen sind besonders geeignet als dentale Abformmassen, Bissregistriermassen, Modellmaterialien, provisorische Verschlussmassen, Materialien zur Herstellung von provisorischen Kronen und Brücken sowie Dubliermassen.

Dabei kann die Dosierung der beiden Komponenten nach Sicht, wie über Stranglängenvergleich, nach Gewicht, über vordosierte Packungseinheiten und nachfolgende Handanmischung, aus Doppelkammerkartuschen mit statischem Mischrohr oder mittels Volumendosieranlagen mit nachgeschaltetem statischem oder dynamischem Mischer erfolgen.

Zur Erzielung optimaler Ergebnisse ist eine hohe Mischgüte erforderlich. Dagegen ist die Toleranz des Mischungsverhältnisses im allgemeinen relativ groß und kann beispielsweise bei einem vorgegebenen Verhältnis von Katalysatorkomponente zu Basiskomponente von 1 : 5 den Bereich 0,8 bis 1,2 : 5 umfassen, ohne dass einsatzbeschränkende Eigenschaftsänderungen feststellbar sind.

Gegenstand der Erfindung sind auch Behältnisse und Mischvorrichtungen, enthaltend die aus den erfindungsgemäßen Zubereitungen hergestellten Massen, insbesondere Dentalmassen, wie Kartuschen, Beutel, Abformlöffel, statische und dynamische Mischer bzw. Mischgeräte.

Die Erfindung wird durch die nachfolgenden Beispiele näher erläutert, ohne dass sie durch diese beschränkt werden soll.

### Beispiele:

### Synthesebeispiel 1

### Darstellung eines Polyether-Silicon-Polyether-Block-Copolymers aus einem H-terminierten Silikonöl und einem Polyethylenglykol-mono-allylether

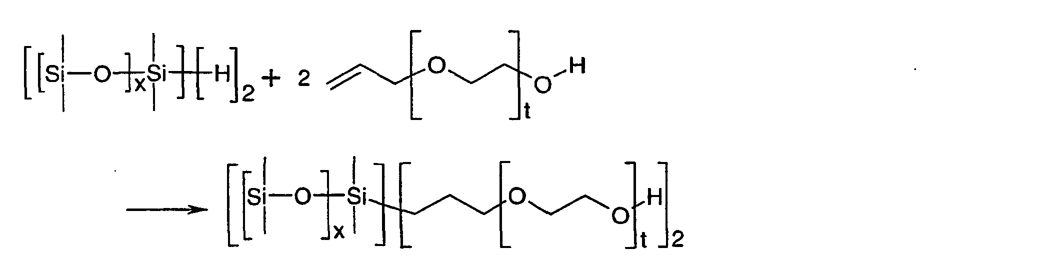

100 g eines Silikonöls DMS-H21 (Gelest) [M = 6000; 0,017 Mol] werden mit 50 ml Toluol, 5 mg Hexachloroplatinsäure in i-Propanol und 15 g Polyethylenglykol-mono-allylether (Clariant) [M = 450; 0,034 Mol] versetzt. Die Mischung wird auf 50° C erhitzt, nach einigen Stunden wird die Temperatur auf 70° C erhöht und solange bei 70° C gehalten, bis die Umsatzkontrolle mittels IR-Spektroskopie das Verschwinden der C=C-Doppelbindung anzeigt. Nach üblicher Aufarbeitung und Produktisolierung verbleiben 110 g eines klaren, farblosen Triblock-Polymers mit der OH-Zahl 16,3.

### Synthesebeispiel 2

### Darstellung eines Acrylat-terminierten Polyether-Silikon-Polyether Blockcopolymers aus dem entsprechenden Blockcopolymer-diol

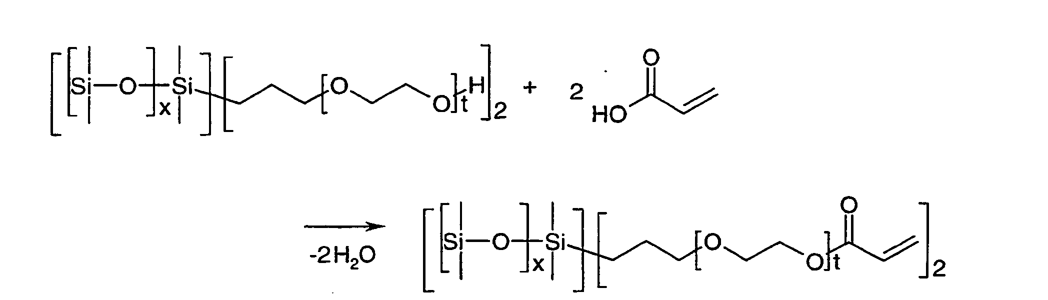

100 g eines Silikonöls aus Synthesebeispiel 1 [OHZ = 16,3; M = 6900; 0,0145 Mol] werden stabilisiert und mit 200 ml Cyclohexan und 4,18 g Acrylsäure [M = 72,06; 0,058 Mol] versetzt. Die Reaktionsmischung wird am Rückfluss gekocht, bis sich kein weiteres Wasser mehr abscheidet und der Rücklauf wasserklar kondensiert. Nach üblicher Aufarbeitung und Produktisolierung verbleiben 98 g eines klaren, farblosen Acrylat-Blockcopolymers.

### Synthesebeispiel 3

### Darstellung eines Aziridino-Block-Copolymers aus einem Acrylat-terminierten Polyether-Silikon-Polyether-Blockcopolymer

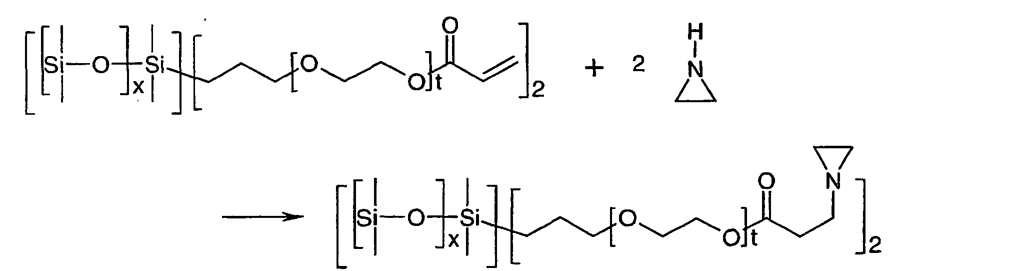

100 g eines Silikonöls aus Synthesebeispiel 2 [M = 7000; 0,0143 Mol] werden mit 50 ml Toluol versetzt. Bei 30° C tropft man 1,3 g Aziridin [M = 43,07; 0,029 Mol] zu. Die Mischung wird auf 50° C erhitzt, nach fünf Stunden wird auf 100° C erhöht und solange bei 100° C gehalten, bis die Umsatzkontrolle mittels IR-Spektroskopie das Verschwinden der Doppelbindung anzeigt. Nach üblicher Aufarbeitung und Produktisolierung verbleiben 100 g eines klaren, farblosen Aziridinosilikons.

### Synthesebeispiel 4:

### Darstellung eines Polyether-Silicon-Polyether-Block-Copolymers aus einem H-terminierten Silikonöl und einem Polyethylenglykol-mono-allylether

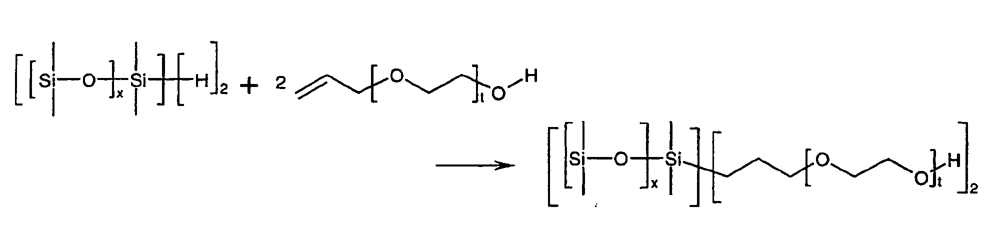

100 g eines Silikonöls Modifier 705 (Hanse Chemie) [M = 12500; 0,08 Mol] werden mit 50 ml Toluol, 5 mg Hexachloroplatinsäure in i-Propanol und 5,6 g Polyethylenglykol-mono-allylether (Clariant) [M = 350; 0,016 Mol] versetzt. Die Mischung wird auf 50° C erhitzt, nach fünf Stunden wird auf 70° C erhöht und solange bei 70° C gehalten, bis die Umsatzkontrolle mittels IR-Spektroskopie das Verschwinden der Doppelbindung anzeigt. Nach üblicher Aufarbeitung und Produktisolierung verbleiben 102 g eines klaren, farblosen Triblock-Polymers mit der OH-Zahl 8,5.

Die Umsetzung in das Aziridinoderivat erfolgte nach Beispiel 13 der US-PS-34 53 242.

### Synthesebeispiel 5:

### Darstellung eines Polyether-Silicon-Polyether-Block-Copolymers aus einem H-terminierten Silikonöl und einem Poly(ethylenglykol-co-propylenglykol)-mono-allylether

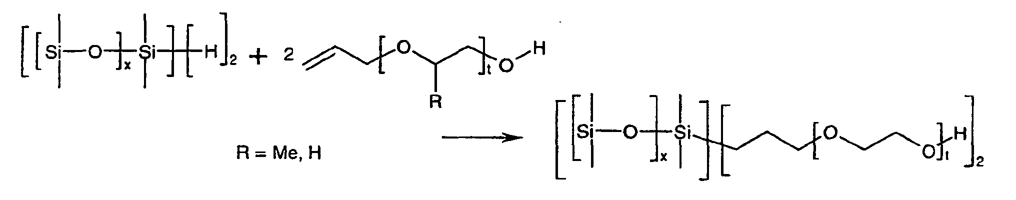

100 g eines Silikonöls DMS-H21 (Gelest) [M = 6000; 0,017 Mol] werden mit 50 ml Toluol, 5 mg Hexachloroplatinsäure in i-Propanol und 68 g Unisafe PKA-5013 (NOF) [M = 2000; 0,034 Mol] versetzt. Die Mischung wird auf 50° C erhitzt, nach fünf Stunden wird auf 70° C erhöht und solange bei 70° C gehalten, bis die Umsatzkontrolle mittels IR-Spektroskopie das Verschwinden der Doppelbindung anzeigt. Nach üblicher Aufarbeitung und Produktisolierung verbleiben 165 g eines leicht trüben, farblosen Triblock-Polymers mit der OH-Zahl 11,2.

Die Umsetzung in das Aziridinoderivat erfolgte nach Beispiel 13 der US-PS-34 53 242.

### Synthesebeispiel 6:

### Darstellung eines Polyether-Silicon-Polyether-Block-Copolymers aus einem H-terminierten Silikonöl und einem Poly(ethylenglykol-co-tetramethylenglykol)-mono-allylether

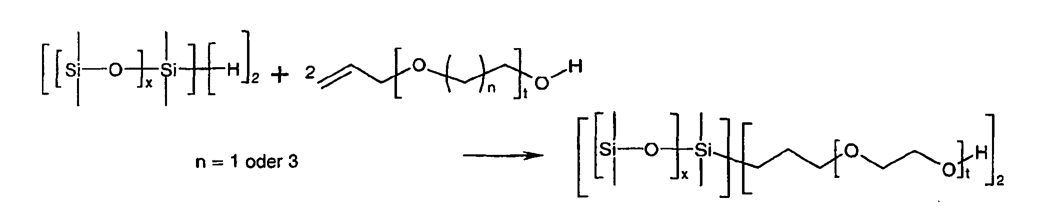

100 g eines Silikonöls DMS-H21 (Gelest) [M = 6000; 0,017 Mol] werden mit 50 ml Toluol, 5 mg Hexachloroplatinsäure in i-Propanol und 81,6 g Unisafe PKA-7302 (NOF) [M = 2400; 0,034 Mol] versetzt. Die Mischung wird auf 50° C erhitzt, nach fünf Stunden wird auf 70° C erhöht und solange bei 70° C gehalten, bis die Umsatzkontrolle mittels IR-Spektroskopie das Verschwinden der Doppelbindung anzeigt. Nach üblicher Aufarbeitung und Produktisolierung verbleiben 178 g eines leicht trüben, farblosen Triblock-Polymers mit der OH-Zahl 10,4.

Die Umsetzung in das Aziridinoderivat erfolgte nach Beispiel 13 der US-PS-34 53 242.

### Herstellungsbeispiele Dentalmassen

Die nachfolgend beschriebenen Katalysatorkomponenten wurden im 100 g-Maßstab hergestellt. Die Herstellung der Basiskomponenten, die in Tabelle 1 beschrieben sind, erfolgte im 500 g-Maßstab.

### Anwendungsbeispiele

### Herstellung der Katalysatorkomponenten

### Anwendungsbeispiel 1 (K1)

In einem Laborkneter wurden 44 g Acetyltributylcitrat gemäß Bestandteil (C) vorgelegt und 20 g β-(S-Lauryl-S-ethylsulfonium)butyronitrilfluoroborat gemäß Bestandteil (B) (s. US-41 67 618) eingelöst. In diese Mischung wurden 12 g Diatomeenerde und 24 g pyrogener Kieselsäure (HDK H 2000, Fa. Wacker), beide gemäß Bestandteil (D) eingearbeitet.

### Anwendungsbeispiel 2 (K2)

In einem Laborkneter wurden 61,1 g eines Poly(-ethylen, -propylen)glykols gemäß Bestandteil (C) mit einer Molmasse von 3400 g / Mol vorgelegt und schrittweise 21 g einer hydrophobierten Fällungskieselsäure gemäß Bestandteil (D) (Sipemat D 17, Fa. Degussa) zugegeben.

9,9 g p-Toluolsulfonsäure-Monohydrat gemäß Bestandteil (B) wurden in 5 g destilliertem Wasser gelöst und der pastenförmigen Mischung zugesetzt. Nach Homogenisierung erfolgte die Zugabe einer Paste bestehend aus 2 g Zinkoxid gemäß Bestandteil (B) und 1 g Poly(-ethylen, -propylen)glykol gemäß Bestandteil (C) mit einer Molmasse von 3400 g / Mol. Die Paste wurde nach der letzten Zugabe noch eine Stunde geknetet.

### Anwendungsbeispiel 3 (K3)

In einem Laborkneter wurden 19 g hydrophobierte Fällungskieselsäure gemäß Bestandteil (D) (Sipernat D 17, Fa. Degussa) in 31 g eines Polypropylenoxid-diol gemäß Bestandteil (C) mit einer Molmasse von 2000 g / Mol eingearbeitet. Dieser pastenförmigen Mischung wurde die Lösung einer Komplexverbindung gemäß Bestandteil (B), hergestellt aus 3,6 g Borsäure und 17 g Salicylalkohol, in 29,4 g Polypropylenoxiddiol gemäß Bestandteil (C) zugegeben und die Paste eine Stunde geknetet.

In Tabelle 2 sind die Mischungen zusammengestellt, die unter Verwendung der beschriebenen Katalysatorkomponenten und der in Tabelle 1 beschriebenen Basiskomponenten im jeweils angegebenen Gewichtsverhältnis untersucht wurden.

Die angegebenen Mischungen wurden durch Anspateln auf den Mischblock innerhalb von 30 Sekunden zubereitet und zur Bestimmung der in Tabelle 2 zusammengestellten Eigenschaften eingesetzt.

**Tabelle 1**

| Zusammensetzung der Basiskomponenten | | | | | | | |
|---|---|---|---|---|---|---|---|
| Verbindung | gemäß Bestandteil | B1 | B2 | B3 | B4 | B5 | B6 |
| | | Gew.-% | | | | | |
| N-Alkylaziridinoblockcopolymer gemäß Synthesebeispiel 3 | (A) | 59,3 | - | - | - | - | 8,3 |
| N-Alkylaziridinoblockcopolymer gemäß Synthesebeispiel 4 | (A) | - | 63,2 | - | - | 50,1 | 55,1 |
| N-Alkylaziridinoblockcopolymer gemäß Synthesebeispiel 5 | (A) | - | - | - | 57,6 | - | - |
| N-Alkylaziridinoblockcopolymer gemäß Synthesebeispiel 6 | (A) | - | - | 54,3 | - | 9,8 | - |
| Dimethylsiloxan AK 50 mit einer Viskosität von 50 mPas | (C) | 10,1 | 4,1 | - | - | 4,9 | 10,0 |
| Dimethylsiloxan AK 5000 mit einer Viskosität von 5000 mPas | (C) | 6,3 | 5,7 | - | - | 9,2 | 2,8 |
| Quarzpulver (Silbond TST 600, Frechen) | (D) | - | - | 25,7 | 34,8 | 24,8 | |
| Diatomeenerde | (D) | 24,3 | 27,0 | 10,0 | - | - | 23,0 |
| Silikon-Polyether-Blockpolymer (Silwet L 7280, Fa. Witco) | (C) | - | - | 1,8 | 2,6 | 1,2 | 0,8 |
| Dibenzyltoluol | (C) | - | - | 2,0 | 5,0 | - | - |
| Gemisch aus Bisaziridinopolyethern mit einer mittleren Imino-äquivalentmasse von 3100, hergestellt aus einem Polyetherdiol, das aus Ethylenoxid und Tetrahydrofuran-Einheiten im Molverhältnis 1 : 3,5 besteht, mit einem Gehalt an cyclischen Polyethern von 0,27 % | - | - | - | 6,2 | - | - | - |

Die erfindungsgemäßen Basiskomponenten gemäß Tabelle 1 zeichneten sich durch eine sehr gute Lagerbeständigkeit aus.

So ließen sich die bei 36° C über eine Zeitraum von 9 Monaten eingelagerten Proben noch einwandfrei mit den angegebenen Katalysatorkomponenten verarbeiten und ergaben mechanische Kennwerte des Elastomerfestkörpers, die um weniger als 15 % von den Werten abwichen, die zu Beginn der Einlagerung gemessen wurden.

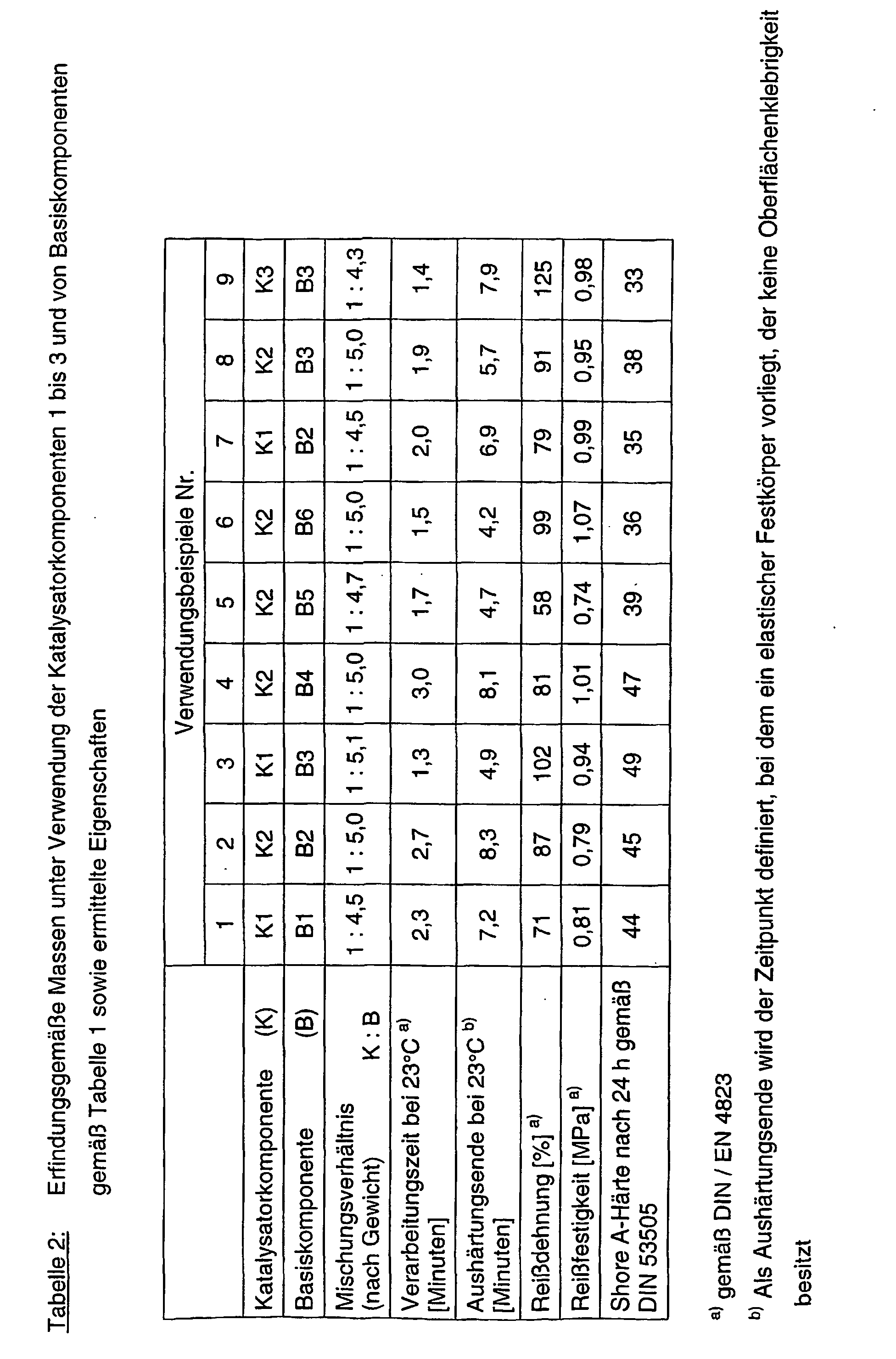

Alle Mischungen der erfindungsgemäßen Verwendungsbeispiele 1 bis 9 (Tabelle 2) erfüllten die Anforderungen an eine elastische Abformmasse und führten zu Formkörpem, die nach einer Lagerzeit bei Raumtemperatur von 24 Stunden eine Shore A-Härte (s. Tabelle 2) deutlich über 20 besaßen.

## Patentansprüche

1. N-Alkylaziridinoblockcopolymere der Struktur: 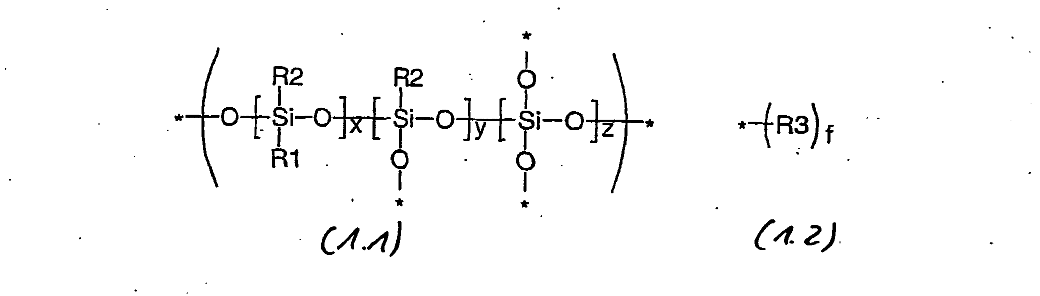 wobei bedeuten:
R1 = H, C₁-C₁₂-Alkyl, C₂-C₁₂-Alkenyl, C₂-C₁₂-Alkinyl, C₇-C₁₅-Alkaryl, C₇-C₁₅₋-Aralkyl oder C₃-C₁₂-Cycloalkyl und diese Reste teilweise, ganz oder gemischt mit Cl oder F substituiert sein können und/oder 0 bis 5 Heteroatome aus der Gruppe O, N, S enthalten können;
R2 = einen Rest aus der Gruppe R1 und/oder R4;
R3 = SiR1₃ oder SiR1₂R4
mit:
R4 = ein Vertreter der allgemeinen Formel (2): 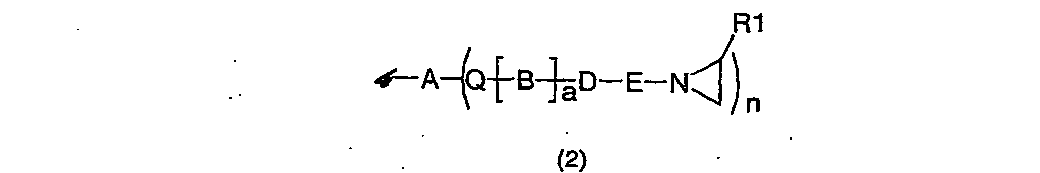 worin:
A = ein (n + 1)-fach radikalischer gesättigter, ungesättigter oder aromatischer, linearer, verzweigter oder cyclischer Kohlenwasserstoffrest, der 0 bis 5 Heteroatome aus der Gruppe O, N, S enthalten kann und 1 bis 18 Kohlenstoffatome umfasst;
B = ein Vertreter der Gruppe: O, S, NR1;
D = ein Vertreter der Gruppe: C(O)O, C(O)NR1, C(O), C(S)NR1, CH₂;
E = ein 2-fach radikalischer gesättigter oder ungesättigter, linearer, verzweigter oder cyclischer Kohlenwasserstoffrest, der 0 bis 5 Heteroatome aus der Gruppe O, N, S, enthalten kann und 0 bis 18 Kohlenstoffatome umfasst;
Q = eine α, ω- radikalische Polyetherkette der allgemeinen Formel (3):
-[O-C_{g}H_{2g}]ₕ- (3)
wobei bedeuten:
g = 2 bis 20;
h = 1 bis 1000;
a = 0 oder 1;
f = eine ganze Zahl von 2 bis 1000;
n = eine ganze Zahl von 1 bis 10;
sowie
x, y, z: jeweils entweder 0 oder ganze Zahlen darstellen, deren Summe zwischen 1 und 10.000 liegen soll,
mit den Einschränkungen, dass, wenn x größer 0 ist, y oder z kleiner- oder gleich x sein soll.
wobei as Symbol "*" in der Formel (1.2) bedeutet, dass die so markierte Valenz mit den mit "*" markierten Positionen des Fragments (1.1) verknüpft wird.

2. N-Alkylaziridinoblockcopolymere nach Anspruch 1, wobei diese mindestens zwei und bis zu 10 N-Alkylaziridinogruppen pro Molekül enthalten.

3. Härtbare Massen auf der Basis von N-Alkylaziridinoblockcopolymeren gemäß einem der Ansprüche 1 oder 2, wobei diese nach der Mischung der Komponenten, jeweils bezogen auf 100 Masseteile, enthalten:
(A) 30 bis 97 Masseteile von mindestens einem N-Alkylaziridinoblockcopolymer mit Molmassen im Bereich von 500 bis 50.000 g / Mol und Aziridinoäquivalentmassen im Bereich von 250 bis 25.000 g / Äquivalent,
(B) 1 bis 10 Masseteile von Startersubstanzen, die geeignet sind, die Aushärtung der N-Alkylaziridinoblockcopolymeren zu bewirken,
(C) 1 bis 35 Masseteile von organischen Verdünnungsmitteln,
(D) 1 bis 50 Masseteile von Modifikatoren.

4. Zweikomponentige Dentalmassen, enthaltend N-Alkylaziridinoblockcopolymere gemäß einem der Ansprüche 1 bis 2, bestehend aus einer Basiskomponente und einer Katalysatorkomponente, wobei die durch homogene Mischung der Katalysatorkomponente und der Basiskomponente erzeugte Zubereitung eine Verarbeitungszeit bei Raumtemperatur zwischen 0,5 bis 10 Minuten besitzt und die Zubereitung in einem Temperaturbereich von 23 bis 36° C innerhalb einer Zeitspanne von einer bis 20 Minuten zu einer elastisch deformierbaren Masse mit einer Shore A-Härte von mindestens 20 aushärtet.

5. Verwendung der N-Alkylaziridinoblockcopolymere gemäß einem der Ansprüche 1 bis 2 für die Herstellung von härtbaren Massen.

6. Verwendung der N-Alkylaziridinoblockcopolymere gemäß einem der Ansprüche 1 bis 2 für die Herstellung von Dentalmassen.

7. Verwendung der Massen nach einem der Ansprüche 3 und 4 zum Verkleben, Beschichten oder Vergießen von Substraten sowie im Dentalbereich, insbesondere als dentale Abformmassen, Bissregistriermassen, Modellmaterialien, provisorische Verschlussmassen, Materialien zur Herstellung von provisorischen Kronen und Brücken sowie Dubliermassen.

8. Behältnis, enthaltend mindestens eine Masse nach mindestens einem der Ansprüche 3 und 4.

## Claims

1. N-Alkylaziridine block copolymers of the structure: 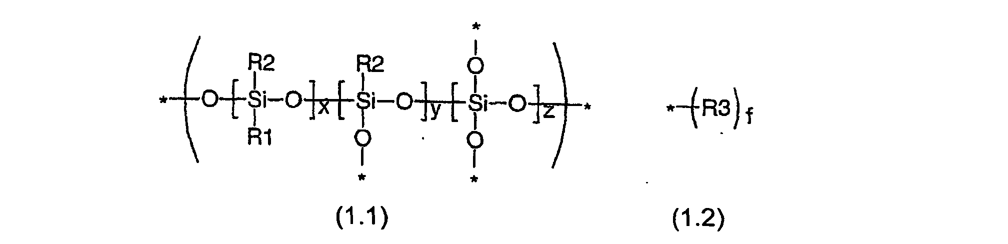 in which
R1 denotes H, C₁-C₁₂-alkyl, C₂-C₁₂-alkenyl, C₂-C₁₂-alkynyl, C₇-C₁₅--alkaryl, C₇-C₁₅-aralkyl or C₃-C₁₂-cycloalkyl and these radicals may be partly or completely substituted by Cl or F or a mixture thereof and/or may contain 0 to 5 hetero atoms from the group consisting of O, N and S;
R2 denotes a radical from the group R1 and/or R4;
R3 denotes SiR1₃ or SiR1₂R4
with:
R4 = a representative of the general formula (2): 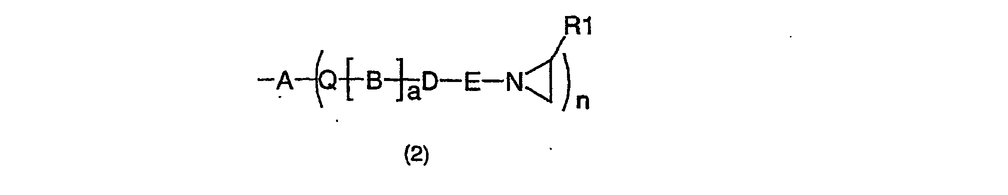
A = a (n + 1)-valent saturated, unsaturated or aromatic, linear, branched or cyclic hydrocarbon radical which contain 0 to 5 hetero atoms from the group consisting of O, N and S and comprises 1 to 18 carbon atoms;may
B = a representative of the group O, S, NR1
D = a representative of the group C(O)O, C(O)NR1, C(O), C(S)NR1 and CH₂;
E = a divalent saturated or unsaturated, linear, branched or cyclic hydrocarbon radical which may contain 0 to 5 hetero atoms from the group consisting of O, N and S and comprises 0 to 18 carbon atoms;
Q = an α,ω-polyether chain of the general formula (3): in which:
-[O-C_{g}H_{2g}]ₕ- (3)
in which
g denotes 2 to 20;
h denotes 1 to 1 000;
a denotes 0 or 1;
f denotes an integer from 2 to 1 000;
n denotes an integer from 1 to 10;
and
x, y and z each represent either 0 or integers whose sum should be between 1 and 10 000,
with the limitations that, if x is greater than 0, y or z should be less than or equal to x,
the symbol "*" in the formula (1.2) meaning that the valency thus marked is linked to those positions of the fragment (1.1) which are marked "*".

2. N-Alkylaziridine block copolymers according to Claim 1, which contain at least two and up to 10 N-alkylaziridino groups per molecule.

3. Curable materials based on N-alkylaziridine block copolymers according to either of Claims 1 and 2, which, after mixing of the components and based in each case on 100 parts by mass, contains:
(A) 30 to 97 parts by mass of at least one N-alkylaziridine block copolymer having a molar mass in the range from 500 to 50 000 g/mol and an aziridino equivalent mass in the range from 250 to 25 000 g/equivalent,
(B) 1 to 10 parts by mass of initiator substances which are suitable for curing the N-alkylaziridine block copolymers,
(C) 1 to 35 parts by mass of organic diluents,
(D) 1 to 50 parts by mass of modifiers.

4. Two-component dental materials containing N-alkylaziridine block copolymers according to either of Claims 1 and 2, consisting of a base component and a catalyst component, the preparation produced by homogeneous mixing of the catalyst component and the base component having a processing time of between 0.5 and 10 minutes at room temperature, and the preparation curing in a temperature range from 23 to 36°C within a time span of one to 20 minutes to give an elastically deformable material having a Shore A hardness of at least 20.

5. Use of the N-alkylaziridine block copolymers according to either of Claims 1 and 2 for the preparation of curable materials.

6. Use of the N-alkylaziridine block copolymers according to either of Claims 1 and 2 for the preparation of dental materials.

7. Use of the materials according to either of Claims 3 and 4 for adhesive bonding, coating or casting of substrates and in the dental sector, in particular as dental impression materials, bite-recording materials, modelling materials, temporary sealing materials, materials for the production of temporary crowns and bridges, and duplication materials.

8. Container containing at least one material according to at least one of Claims 3 and 4.

## Revendications

1. Copolymères à blocs N-alkylaziridino présentant la structure : 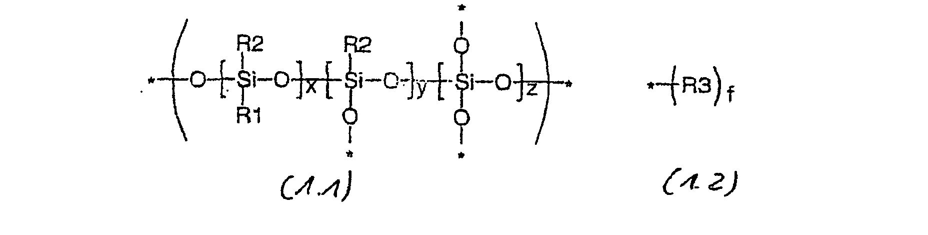 dans laquelle
R1 = H, alkyle en C₁ à C₁₂, alcényle en C₂ à C₁₂, alcynyle en C₂ à C₁₂, alkaryle en C₇ à C₁₅, aralkyle en C₇ à C₁₅ ou cycloalkyle en C₃ à C₁₂ et ces radicaux peuvent être substitués partiellement, complètement ou en mélange par Cl ou F et/ou contenir 0 à 5 hétéroatomes du groupe O, N, S ;
R2 = un radical du groupe R1 et/ou R4 ;
R3 = SiR1₃ ou SiR1₂R4
avec
R4 = un représentant de formule générale (2) : 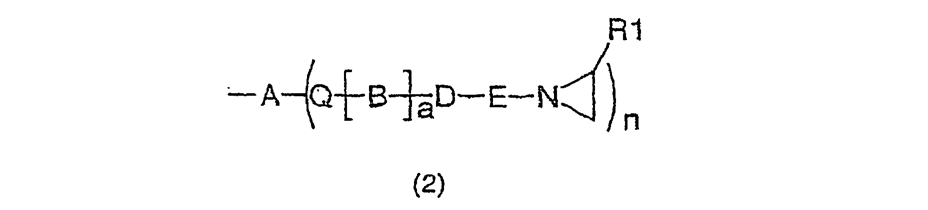 où
A = radical hydrocarboné radicalaire (n+1)valent, saturé, insaturé ou aromatique, linéaire, ramifié ou cyclique, qui peut contenir 0 à 5 hétéroatomes du groupe O, N, S et qui comprend 1 à 18 atomes de carbone ;
B = un représentant du groupe : O, S, NR1 ;
D = un représentant du groupe : C(O)O, C(O)NR1, C(O), C(S)NR1, CH₂ ;
E = un radical hydrocarboné radicalaire divalent, saturé ou insaturé, linéaire, ramifié ou cyclique, qui peut contenir 0 à 5 hétéroatomes du groupe O, N, S et qui comprend 0 à 18 atomes de carbone ;
Q = une chaîne polyéther α,ω-radicalaire de formule générale (3) : 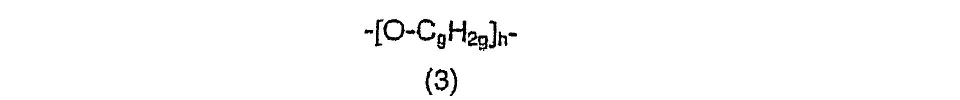 dans laquelle
g=2à20;
h=1 à 1000 ;
a = 0 ou 1 ;
f = un nombre entier de 2 à 1000 ;
n = un nombre de 1 à 10 ;
ainsi que
x, y, z : représentent à chaque fois 0 ou des nombres entiers, dont la somme doit être comprise entre 1 et 10000,
avec comme limitations que lorsque x est supérieur à 0, y ou z doit être inférieur ou égal à x,
le symbole "*" dans la formule (1.2) signifiant que la valence ainsi marquée est liée avec les positions repérées par "*" du fragment (1.1).

2. Copolymères à blocs N-alkylaziridino selon la revendication 1, ceux-ci contenant au moins deux et jusqu'à 10 groupes N-alkylaziridino par molécule.

3. Masses durcissables à base de copolymères à blocs N-alkylaziridino selon l'une quelconque des revendications 1 ou 2, celles-ci contenant après le mélange des composants, à chaque fois par rapport à 100 parties en masse :
(A) 30 à 97 parties en masse d'au moins un copolymère à blocs N-alkylaziridino présentant des masses molaires dans la plage de 500 à 50000 g/mole et des masses d'équivalents aziridino dans la plage de 250 à 25000 g/équivalent
(B) 1 à 10 parties en masse d'initiateurs, qui conviennent pour provoquer le durcissement des copolymères à blocs N-alkylaziridino,
(C) 1 à 35 parties en masse de diluants organiques,
(D) 1 à 50 parties en masse de modificateurs.

4. Masses dentaires à deux composants, contenant des copolymères à blocs N-alkylaziridino selon l'une quelconque des revendications 1 à 2, constituées par un composant de base et un composant catalyseur, la préparation obtenue par le mélange homogène du composant catalyseur et du composant de base présentant un temps de transformation à température ambiante entre 0,5 à 10 minutes et la préparation ainsi préparée durcissant, dans une plage de température de 23 à 36°C, en un laps de temps de une à 20 minutes en une masse pouvant être déformée élastiquement présentant une dureté Shore A d'au moins 20.

5. Utilisation des copolymères à blocs N-alkylaziridino selon l'une quelconque des revendications 1 à 2 pour la préparation de masses durcissables.

6. Utilisation des copolymères à blocs N-alkylaziridino selon l'une quelconque des revendications 1 à 2 pour la préparation de masses dentaires.

7. Utilisation des masses selon l'une quelconque des revendications 3 et 4 pour le collage, le revêtement et le moulage de substrats ainsi que dans le domaine dentaire, en particulier comme masses dentaires de moulage, masses dentaires d'empreinte, matériaux dentaires pour gabarits, masses dentaires d'obturation provisoires, matériaux dentaires pour la réalisation de couronnes et de bridges provisoires ainsi que masses dentaires de placage.

8. Récipient, contenant au moins une masse selon au moins l'une quelconque des revendications 3 et 4.
